# EUROPEAN PATENT APPLICATION

(11) **EP 1 189 051 A1**
(43) Date of publication of application: **20.03.2002**
(21) Application number: 01904456.9
(22) Date of filing: 15.02.2001
(51) Int. Cl.: G01N 3/12

(54) **COMPRESSION TESTER**

(30) Priority: 25.02.2000 JP 2000049428
(71) Applicant: NGK INSULATORS, LTD., Nagoya-City, Aichi Prefecture 467-8530 (JP)
(72) Inventor: SHIMADA, Shinichi, Nagaya-city, Aichi 467-8530 (JP); NARUSE, Shinichi, B-1170 Brussels (BE); TERAGUCHI, Takashi, Nagoya-city, Aichi 467-8530 (JP)
(74) Representative: Paget, Hugh Charles Edward
(86) International application number: JP0101061
(87) International publication number: WO0163242

(57) **Abstract**

An inspection machine by compression for inspecting a ceramic sample (1), in which the ceramic sample (1) is disposed in a cylindrical container (8) via an elastic sheet (9), and the ceramic sample (1) is compressed by a hydrostatic-pressure-applying medium injected between the cylindrical container (8) and the elastic sheet (9). A rubber material having a thickness of 5mm or less and a hardness of 30 ― 50° is employed as the elastic sheet (9). Since the elastic sheet interposed was made thin and given a predetermined hardness to eliminate pressure decrease almost completely, pressurization can be conducted with high accuracy even under low pressure.

## Description

### Technical Field

The present invention relates to an inspection machine by compression for ceramic samples, and in particular, to an inspection machine by compression for ceramic samples, capable of inspecting with high accuracy down to low pressure.

### Background Art

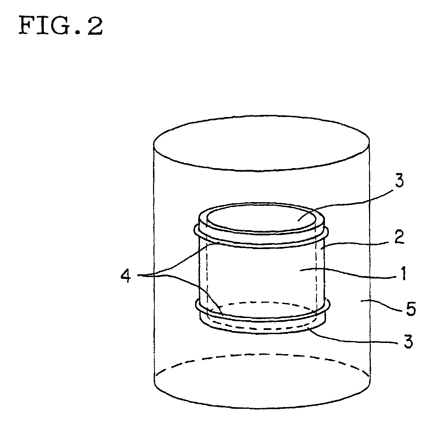

Inspection by compression on a ceramic structure, such as a honeycomb structure, is performed by applying hydrostatic pressure on the ceramic structure. Fig. 2 is a perspective view of a conventional inspection method by compression having a configuration in which a sample 1, disposed in a urethane rubber cylinder 2 having an internal diameter corresponding to the diameter of the sample 1 and having a thickness of 0.5 to 1.0 mm, is provided with disk-like acrylic plates 3 abutted on the sample 1 at the ends thereof, the acrylic plates 3 are fixed to the urethane rubber cylinder 2 by elastic bands 4, and the sample 1 covered by the urethane rubber cylinder 2 and the acrylic plates 3 is placed in water in a tank 5, whereby pressure is applied to the sample 1.

However, a problem in the above-described conventional inspection method by compression has been found to be that the efficiency of operation decreases when many samples are inspected because it is time-consuming to fix the acrylic plates 3 to the urethane rubber cylinders 2 by the elastic bands 4. Another problem is that it is difficult to clean the inspection device after inspecting when wet fractions of samples, which have been broken by the pressure, adhere to the urethane rubber cylinders.

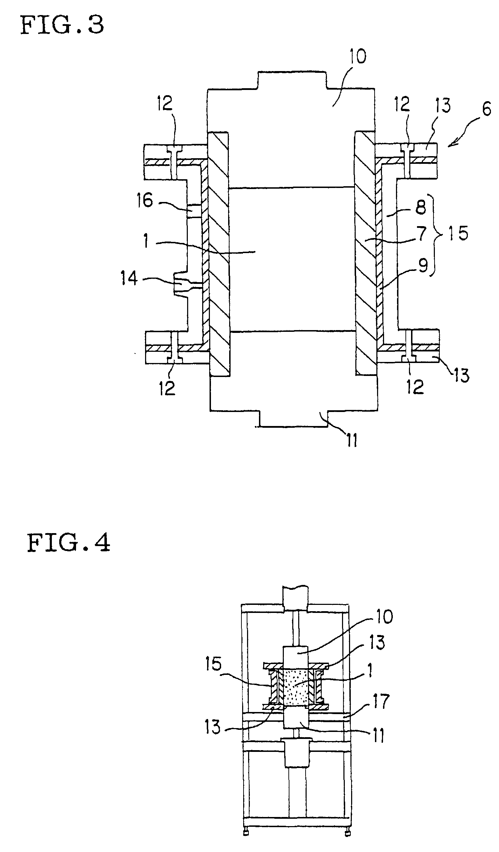

In order to overcome these problems, an inspection machine by compression is disclosed in, for example, Japanese Patent Application Laid-Open No. 10-197429, in which the inspection machine is provided with a cylindrical container, a urethane sleeve, and a urethane sheet. Inspecting using this inspection machine by compression 6 is performed as follows. Referring to Fig. 3, a sample 1 provided with a urethane sleeve 7 around the same is placed in a cylindrical container 8 with a urethane sheet 9 disposed between the urethane sleeve 7 and the cylindrical container 8. Then, a hydrostatic-pressure-applying medium is injected between the cylindrical container 8 and the urethane sheet 9, thereby compressing the sample 1 at the periphery thereof, whereby the inspection by compression is performed.

In this inspection machine by compression, the sample 1 is not fixed by elastic bands. Therefore, the efficiency of operation is not decreased even when many samples are processed. Moreover, after inspecting, dry fractions of the sample 1 remain in the urethane sleeve 7. Therefore, cleaning of the inspection machine is easier than when using the urethane rubber cylinder shown in Fig. 2.

However, a problem in the inspection machine by compression 6 shown in Fig. 3 is that it is difficult to control the pressure applied to the sample 1, because the compression of the sample 1 does not properly respond to the pressure applied to the pressure-applying medium due to the elasticity of the urethane sleeve 7.

In addition, in recent years, a wall of a honeycomb structure becomes thinner and thinner, and a pressure for the inspection is as low as less than 1 MPa. However, in the inspection machine by compression 6 shown in Fig. 3, there was a problem that it is difficult to apply a low pressure to the sample 1 due to the elasticity of the urethane sleeve 7. That is, when the pressure on the sample 1 is desired to be at a pressure of less than 1 MPa, the pressure applied to the pressure-applying medium serves only to compress and deform the urethane sleeve 7 and does not compress the sample 1.

Further, in the inspection machine by compression 6 shown in Fig. 3, the preparations for the inspection are laborious, in which each sample must be inserted to the urethane sleeve 7, then must be disposed in the cylindrical container 8.

The present invention has been made in view of such a situation, and it is an object of the present invention to provide an inspection machine by compression in which application of a lower pressure is possible and inspection is easily conducted with high accuracy.

### Disclosure of Invention

That is, according to the present invention, there is provided an inspection machine by compression for inspecting a ceramic sample, in which the ceramic sample is disposed in a cylindrical container via an elastic sheet, and the ceramic sample is compressed by a hydrostatic-pressure-applying medium injected between the cylindrical container and the elastic sheet, wherein a rubber material having a thickness of 5mm or less and a hardness of 30 - 50° is employed as the elastic sheet.

The above inspection machine by compression for inspecting a ceramic sample can be preferably used even when a pressure applied by the hydrostatic-pressure-applying medium is less than 1 MPa. The elastic sheet more preferably has a thickness of 0.3 - 5 mm, and particularly preferably 0.5 - 2.0 mm. The elastic sheet more preferably has a hardness of 35 ― 45° .

Further, it is preferable that air of a compressible fluid is employed as the hydrostatic-pressure-applying medium because of easy treatment upon breakage of the elastic sheet and of a large filling capacity. It is preferable in this inspection machine by compression for inspecting a ceramic sample that the pressure is raised up to 0.5 MPa for 0.5 ― 10 seconds. This pressurization speed enables to plan to prevent the elastic sheet from breaking by feeding back a fracture detection signal in order to suspend pressurizing. In the inspection machine by compression for inspecting a ceramic sample of the present invention, the ceramic sample may be of a honeycomb structure.

### Brief Description of Drawings

Fig. 1 is a schematic section of an inspection machine by compression according to an embodiment of the present invention.
Fig. 2 is a perspective view of a known inspection machine by compression;
Fig. 3 is a schematic section of another known inspection machine by compression; and
Fig. 4 is a schematic section of the inspection machine by compression according to the present invention, by the use of which an inspection is performed.

### Best Mode for Carrying Out the Invention

The present invention is described in more detail with reference to the embodiments shown in the drawings.

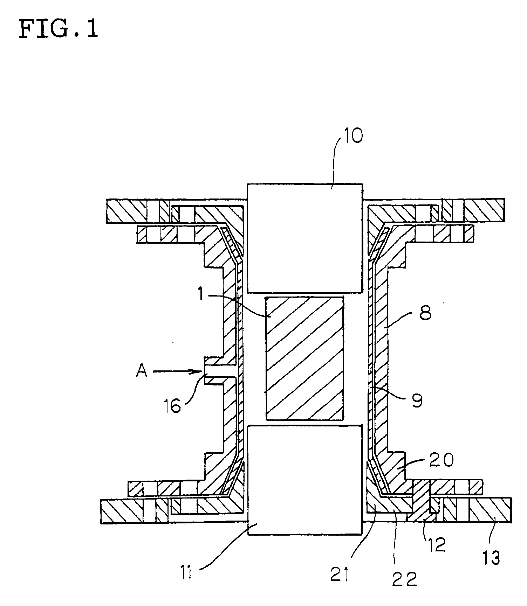

In an inspection machine by compression according to an embodiment of the present invention, a ceramic sample is disposed in a cylindrical container via an elastic sheet, and a hydrostatic-pressure-applying medium is injected between the cylindrical container and the elastic sheet for pressurization, whereby an inspection by compression is performed on the ceramic sample. As shown in Fig. 1, a sample 1 is disposed in an inspection container 15 wherein an elastic sheet 9 is disposed on the internal wall. That is, since the cylindrical container 8 and the elastic sheet 9 unitarily form an inspection container 15, and the elastic sheet 9 adheres to the sample 1 according to the shape of the sample 1 when pressure is applied; it is not necessary to use the conventional urethane sleeve 7 having a shape corresponding to that of a product. Therefore, this is prepared in a manner simpler than in a conventional inspecting machine by compression shown in Fig. 3. In the inspecting machine by compression according to the embodiment of the invention, the pressure rise is suspended by detecting a fracture sound from the inspecting sample; therefore, fractions of the sample can be easily cleaned and the elastic sheet itself is prevented from breaking due to a pressure by the fractions of the sample.

In an inspecting machine by compression of the present invention, only the elastic sheet 9 is interposed between the sample 1 and a pressure-applying medium A, pressure decrease is very small with respect to pressurization, and therefore, pressurization can be freely manipulated. That is, the elastic sheet 9 adheres to the sample 1 due to elasticity (contractibility) of the elastic sheet 9 just after the pressurization is started, and the controlled pressure can directly be applied to the sample 1. In addition, since the pressure applied is directly controlled, problems of fracture of the sample due to a pressure above the predetermined pressure and of an effluence of rejected articles due to a pressure below the predetermined pressure can be avoided.

In an inspecting machine by compression of the present invention, a rubber material having a thickness of 5 mm or less and a hardness of 30 ― 50° as the elastic sheet for pressurizing the sample by a pressure-applying medium is used.

Thus, since a specific rubber material having a thickness of 5 mm or less and a hardness of 30 ― 50° as the elastic sheet is used in the present invention, there is almost no pressure decrease upon pressurization by a pressure-applying medium, and the elastic sheet can adhere to the ceramic sample 1 according to the shape of the sample 1. Therefore, controlled pressure can directly be applied to the sample.

The elastic sheet more preferably has a thickness of 0.3― 5 mm, and particularly preferably 0.5 ― 2.0 mm; and more preferably has a hardness of 35 ― 45° in view of its contractibility and durability. Incidentally, hardness of the elastic sheet was measured on the basis of the hardness test in "Method of physical test of vulcanized rubber" shown in JIS K 6301.

As a material for the elastic sheet, there may be used various kinds of rubber materials such as urethane, silicone, natural rubber, and synthetic rubber. Among them, natural rubber can be preferably used in view of strength against a cut or breakage and low cost.

In addition, as a pressure-applying medium in an inspecting machine by compression of the present invention, both an uncompressible fluid such as water and a compressible fluid such as air may be used. However, it is preferable to use air because of easy treatment upon breakage of the elastic sheet and of a large filling capacity because pressurization is conducted under low pressure in the present invention.

Though pressurization may be conducted in a step since pressure decrease is very small with respect to pressurization as mentioned above in an inspecting machine by compression of the present invention, pressurization may be conducted also in multi-steps consisting of two or more steps.

An inspection machine by compression according to the present invention preferably determines the start of fracture of the sample by detecting a fracture sound from the ceramic sample, thereby suspending pressurizing. The sample is prevented from being unnecessarily broken by suspending pressurizing when the start of fracture of the sample is detected either by detecting a fracture sound at the beginning of fracture of the ceramic sample by an acoustic emission (AE) sensor, or by detecting the change in pressure (change in hydrostatic pressure) inside a cylindrical container due to the fracture of the ceramic sample, whereby excessive fracturing of the sample does not occur, thereby making cleaning easier.

Incidentally, by controlling pressurization speed so as to raise the pressure up to a pressure of 0.5 MPa in 0.5 ― 10 seconds in this inspection machine by compression according to the present invention, a fracture detection signal is fed back for suspending pressurization so that the elastic sheet is prevented from breaking due to a contact with fractions even in the case of using air of a compressible fluid.

There is no limitation on the number of inspection containers disposed in an inspection machine by compression according to the present invention. However, in the case that two inspection containers are provided, samples are put in the two inspection containers, and simultaneously a pressurization inspection is conducted; the sample to be subsequently put in can be prepared during the pressurization, and the operator can effectively work with his position being fixed.

The inspection machine by compression according to the present invention is preferably used for an inspection by compression for ceramic samples, in particular, for an inspection by compression of ceramic honeycomb structures.

The present invention is described hereinbelow with reference to Examples. However, the present invention is by no means limited to these Examples.

### ( Example 1 )

As shown in Fig. 1, a compression inspection of a ceramic honeycomb structure was performed by using an inspection machine by compression 6 including a cylindrical container 8, a urethane sheet 9 as the elastic sheet, and an inspection container 15.

The cylindrical container 8 made of iron was provided with the urethane sheet 9 having a thickness of 1 mm inside the cylindrical container 8. Each of both end portions of the urethane sheet 9 was put between a tapered portion 20 arranged in both end portions of the cylindrical container 8 and a mount 22 provided with a tapered portion 21 having a shape corresponding with the tapered portion 20. The cylindrical container 8 was fixed to the mount 22 by means of a bolt 12 to maintain airtightness. Incidentally, the mount 22 has a structure of communicating with a flange 13, and the cylindrical container 8 is provided with a pressure inlet 16 connectable with a pressure hose of a compressing unit (not shown).

On the occasion of inspecting, the inspecting container 15 was fixed to a base 17, as shown in Fig. 4. A supporting cylinder 11 was moved to the upper level of the cylindrical container 15, a honeycomb structure 1 was mounted on the supporting cylinder 11, the supporting cylinder 11 was moved down to the lower level of the inspecting container 15, and the honeycomb structure 1 was thus disposed in the inspecting container 15. A retaining cylinder 10 was disposed on the honeycomb structure 1, and the supporting cylinder 11 and the retaining cylinder 10 clamped the honeycomb structure 1 with a force of approximately 1 MPa so that the honeycomb structure 1 did not move with the pressure applied thereto.

Next, the compressing unit (not shown) started pressurization through the pressure inlet 16 connecting with the pressure hose. First, pressurization was conducted up to 0.7 MPa for 2 seconds. Soon after the pressurization, the urethane sheet 9 adhered to the periphery of the honeycomb structure 1. Pressure was uniformly applied on the entire periphery of the honeycomb structure 1 via the urethane sheet 9. Fig. 4 shows conditions of the inspection machine by compression 6 while performing the inspection. In the inspection, the honeycomb structure 1 was fractured when the pressure rose to 0.5 MPa; therefore, the pressurization was suspended, and the pressure was reduced. The fracture was detected by detecting the change in pressure (decrease in pressure) inside the inspection container 15.

### Industrial Applicability

According to an inspection machine by compression of the present invention, an elastic sheet interposed between a pressure-applying medium and a sample to be inspected was made thin and given a predetermined hardness to eliminate pressure decrease almost completely; thereby pressurization can be conducted with high accuracy even under low pressure. In addition, pressure can be easily adjusted since the controlled pressure can be directly applied in that case.

Further, if the start of fracture of the sample is detected either by detecting the change in pressure inside a cylindrical container or by detecting a fracture sound of the ceramic sample to suspend pressurization in an inspection machine by compression of the present invention; fracture of the sample can be minimized, the elastic sheet itself is prevented from breaking, and fractions of the sample can be more easily cleaned.

## Claims

1. An inspection machine by compression for inspecting a ceramic sample, in which the ceramic sample is disposed in a cylindrical container via an elastic sheet, and the ceramic sample is compressed by a hydrostatic pressure-applying medium injected between the cylindrical container and the elastic sheet,
wherein a rubber material having a thickness of 5mm or less and a hardness of 30 ― 50° is employed as the elastic sheet.

2. An inspection machine by compression for inspecting a ceramic sample according to Claim 1, wherein a pressure applied by the hydrostatic-pressure-applying medium is less than 1 MPa.

3. An inspection machine by compression for inspecting a ceramic sample according to Claim 1 or 2, wherein said elastic sheet has a thickness of 0.3 ― 5 mm.

4. An inspection machine by compression for inspecting a ceramic sample according to any one of Claims 1 - 3, wherein air of a compressible fluid is employed as the hydrostatic-pressure-applying medium.

5. An inspection machine by compression for inspecting a ceramic sample according to any one of Claims 1 ― 4, wherein the pressure is raised up to 0.5 MPa for 0.5 - 10 seconds.

6. An inspection machine by compression for inspecting a ceramic sample according to any one of Claims 1 ― 5, wherein the ceramic sample is of a honeycomb structure.
